# EUROPEAN PATENT APPLICATION

(11) **EP 4 311 499 A1**
(43) Date of publication of application: **31.01.2024**
(21) Application number: 22187012.4
(22) Date of filing: 26.07.2022
(51) Int. Cl.: A61B 8/08, A61B 8/00

(54) **ULTRASOUND IMAGE ACQUISITION**

(71) Applicant: Koninklijke Philips N.V., 5656 AG Eindhoven (NL)
(72) Inventor: GOOßEN, André, Eindhoven (NL); GESSERT, Nils Thorben, Eindhoven (NL); LOSSAU, Tanja, 5656AG Eindhoven (NL); WEBER, Frank Michael, 5656AG Eindhoven (NL); WAECHTER-STEHLE, Irina, Eindhoven (NL); WEHLE, Simon, Eindhoven (NL); PETERS, Jochen, Eindhoven (NL); WILD, Sebastian, Eindhoven (NL)
(74) Representative: Philips Intellectual Property & Standards

(57) **Abstract**

A method and system for automatically detecting within an input 3D ultrasound image one or more suspected disease features and automatically computing an optimum one or more planes through the 3D field of view for acquiring imagery that would best assist in confirming or further analyzing the suspected disease features. The determined optimum one or more planes are used to control acquisition by an ultrasound acquisition system of new 2D images which correspond to said determined optimum planes.

## Description

### FIELD OF THE INVENTION

This invention relates to ultrasound image acquisition.

### BACKGROUND OF THE INVENTION

In traditional ultrasound examination, the sonographer must manually decide as they carry out the examination one or more planes which are to be recorded. Planes are recorded for the purposes of diagnostics or for evidencing a diagnosis which the sonographer performs in real time as they look at the images. Planes might also be recorded for performing measurements of certain anatomical features.

Each additional plane recorded is time-consuming. Within the art there are known systems which automatically record certain standard planes for standardized procedures without the need for a sonographer to explicitly reconfigure the protocol or reposition the transducer. This is achieved by locating the target anatomy within the 3D field of view (FOV), e.g., by model-based segmentation, and deriving slices through that segmented anatomy that are acquired using dedicated beam-forming protocols. Reference is made for example to the patent applications: WO 2015/087218 and WO 2014/162232.

### SUMMARY OF THE INVENTION

In the context of an examination for the purposes of disease screening or diagnosis, the acquisition of planes is guided by the sonographer's judgment in identifying suspicious features in the images being acquired in real time.

Especially for less trained sonographers, incidental findings or suspicious regions might be overlooked. If this happens, diagnosis can be imprecise or, in a worst-case scenario, critical conditions might be missed. As each additional acquisition is time-consuming and prolongs the exam time, the sonographer must strike a balance between short exam time and potentially missing an important finding, while at the same time always acquiring the maximum number of views to make sure every potential finding is covered. In most practical clinical situations the sonographer would lean in favor shorter exam times due to lack of capacity and for the reason that incidental findings occur with low probability. However, this leaves open the risk of missing a potentially important disease indicator.

The invention is defined by the claims.

According to examples in accordance with an aspect of the invention, there is provided a computer-implemented method, comprising:
receiving as input from an ultrasound acquisition system a 3D ultrasound image dataset comprising at least one 3D image frame spanning a 3D field;
applying a disease detection module adapted for processing input 3D image frames and detecting one or more suspected disease features therein of a pre-defined set of possible disease features;
applying a plane-mapping module adapted to determine for each 3D image frame a set of one or more 2D slices through the 3D field of the image frame based on an output from the disease detection module, for imaging one or more disease regions associated with the detected one or more disease features; and
generating control instructions for output to the ultrasound acquisition system for causing automated acquisition of the set of one or more 2D slices, for example using 2D imaging, for example using B-mode imaging.

Thus, embodiments of the invention are based on the concept of automatically detecting suspected disease features within acquired 3D images using a disease detection module and, in addition, automatically determining optimum image acquisition instructions for acquiring new 2D image frames which capture one or more planes which provide a best representation of the anatomy for analyzing the disease feature(s) for the purpose of diagnosis. By controlling acquisition of new 2D images, and not for example simply extracting slices from the already acquired 3D images, so higher resolution 2D frames of the relevant regions can be acquired, or frames which otherwise have their acquisition parameters/properties optimized for subsequent diagnosis. The acquired 3D image stream may be of lower quality or resolution, suitable for surveying the anatomical region but potentially not of sufficient quality for performing the final diagnostic analysis. Furthermore, by automatically determining the optimum 2D image planes which are to be captured, the user does not need to make this judgment, and the risk of potentially missed findings is reduced.

In some embodiments, the input 3D ultrasound image dataset comprises a stream of 3D image frames, and wherein the steps of the method are performed in real time with receipt of each 3D image frame.

In some embodiments, the plane-mapping module is adapted to use a look-up table to select a pre-determined plane based on the one or more detected disease features. The plane may for example be a plane which is pre-determined as best suited for clinical evaluation of the given detected anatomical feature.

In some embodiments, the disease detection module is adapted to generate for the at least one 3D image frame a 3D spatial map of disease regions within the 3D field of the image frame corresponding to the disease features.

In some embodiments, the plane-mapping module is adapted for receiving as input a 3D map of detected disease regions within a 3D field, and is adapted for determining a set of one or more 2D slices through the 3D field intersecting with the disease regions, in dependence upon the map.

In some embodiments, the plane mapping module is adapted to perform a spatial fitting of planes to the one or more disease regions, to determine a set of one or more 2D slices which intersect all of the regions, and optionally which meet a further one or more constraints.

With reference to the further one or more constraints, these may depend in part on the specific disease features to which the disease regions correspond.

The further one or more constraints may comprise a requirement to minimize the number of 2D slices, while still intersecting all of the regions.

The further one or more constraints may comprise one or more allowable ranges for 2D slice plane orientation relative to one or more directions.

In some embodiments, for at least a subset of the pre-defined set of disease features, the detection of the disease feature by the disease-detection module comprises segmenting and classifying one or more spatial regions as suspicious regions.

In cases where the disease detection module is adapted to generate a 3D spatial map, then the 3D spatial map output by the disease detection module may comprise a map of the segmented suspicious regions.

In some embodiments, for at least a subset of the pre-defined set of disease features, the detection of the respective disease feature by the disease-detection module comprises computing a 3D saliency map spanning the 3D field in relation to the disease feature, and deriving a discrete classification of the 3D image in relation to the feature based on the saliency map. In cases where the disease detection module is adapted to generate a 3D spatial map, then the 3D spatial map output by the disease detection module may be the saliency map. In other words, the saliency map is used as the 3D spatial map of disease regions. For example, a machine learning algorithm which is trained to generate one or more disease feature classifications in relation to an input image frame may generate a 3D saliency map indicative of saliency of different image locations to the final classification. In some embodiments, the plane mapping module is adapted to determine the set of one or more 2D slices based on fitting planes of maximum saliency through the saliency map. A plane of maximum saliency means for example a plane which plots a planar path of maximum saliency, i.e. a plane which contains maximum aggregate saliency values.

In some embodiments, the new 2D images may be generated in the background of a continuing image acquisition procedure being performed by a user using the ultrasound acquisition apparatus. For example, in some embodiments, the control instructions are adapted to cause the ultrasound acquisition system to interleave acquisition of the set of one or more 2D slices with any other acquisition sequence which the ultrasound acquisition system is currently performing.

In some embodiments, the disease detection module comprises at least one trained machine learning algorithm, and preferably a convolutional neural network (CNN).

As noted briefly above, in some embodiments, the control instructions are adapted to control acquisition of 2D slices which are of higher spatial resolution than the input 3D ultrasound image data.

In some embodiments, the method further comprises receiving the acquired set of one or more 2D slices and controlling a user interface to generate a visual output representative thereof.

In some embodiments, the method further comprises, after determining the set of 2D slices, controlling a user interface to generate a user-perceptible prompt requesting approval to acquire the set of 2D slices, and wherein the generating of the control instructions is performed only responsive to receipt from the user interface of a user input indicative of approval.

The invention can also be embodied in hardware.

Thus, another aspect of the invention is a processing device comprising: an input/output; and one or more processors. The one or more processors are configured to perform a method comprising: receiving at the input/output, as input from an ultrasound acquisition system, a 3D ultrasound image dataset comprising at least one 3D image frame spanning a 3D field; applying a disease detection module adapted for processing input 3D image frames and detecting one or more suspected disease features therein of a pre-defined set of possible disease features; applying a plane-mapping module adapted to determine a set of one or more 2D slices through the 3D field of each image based on an output from the disease detection module, for imaging one or more disease regions associated with the detected one or more disease features; and generating control instructions for output via the input/output to the ultrasound acquisition system for causing automated acquisition of the set of one or more 2D slices, for example using a 2D imaging mode, for example using B-mode imaging.

Another aspect of the invention is a system which comprises: an ultrasound acquisition system; and a processing device as recited above, or in accordance with any embodiment described in this document, the processing device operatively coupled to the ultrasound acquisition system. The system may in some embodiments further comprise a user interface device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components and a processing flow of an example processing device in accordance with one or more embodiments of the invention;
Fig. 3 outlines steps of an example method in accordance with one set of embodiments; and
Fig. 4 outlines steps of an example method in accordance with one set of embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for automatically detecting, within an input 3D ultrasound image, one or more suspected disease features and automatically computing an optimum one or more planes through the 3D field of view for acquiring imagery that would best assist in confirming or further analyzing the suspected disease features. The determined optimum one or more planes are used to control acquisition by an ultrasound acquisition system of new 2D images which correspond to said determined optimum planes.

The new 2D images can preferably be acquired with a higher spatial resolution than the original 3D image frames, thus allowing for further diagnostic analysis of the suspected disease features using imagery which is of higher quality.

The ultimate goal is for example to automatically record best-suited slices for evaluation of incidental findings or suspicious regions.

In accordance with advantageous embodiments, it is proposed to add a disease detection module into the image processing pipeline that is adapted to (for example continuously) scan all acquired 3D frames for suspicious features or abnormalities. From this module, optionally a spatial map of anatomical regions pertaining to these features could be derived. This map could then be fed into an optimization step to compute an optimum one or more 2D slices incorporating the mapped regions in order to confirm the finding. The latter step could be performed by a dedicated module, which will be referred to in this disclosure as a plane mapping module.

The determined optimal 2D slices can then be automatically recorded by reconfiguring the transducer arrangement of the ultrasound acquisition system, ideally without the sonographer even taking notice, e.g., by using scan pauses or interleaved scanning. Recorded slices can optionally be further processed with a validation module or a plane predictor module to confirm whether the acquired slice actually corresponds to the target plane, i.e. if the target slice has been hit or if further optimization needs to be performed until convergence.

Finally, additionally recorded slices can be visualized to the sonographer either: after notification, on demand, upon finalizing the exam, or upon reviewing the exam data at a later time.

The above represent features of an example set of embodiments, and not all features mentioned are essential to the inventive concept, as will become clear in forthcoming descriptions.

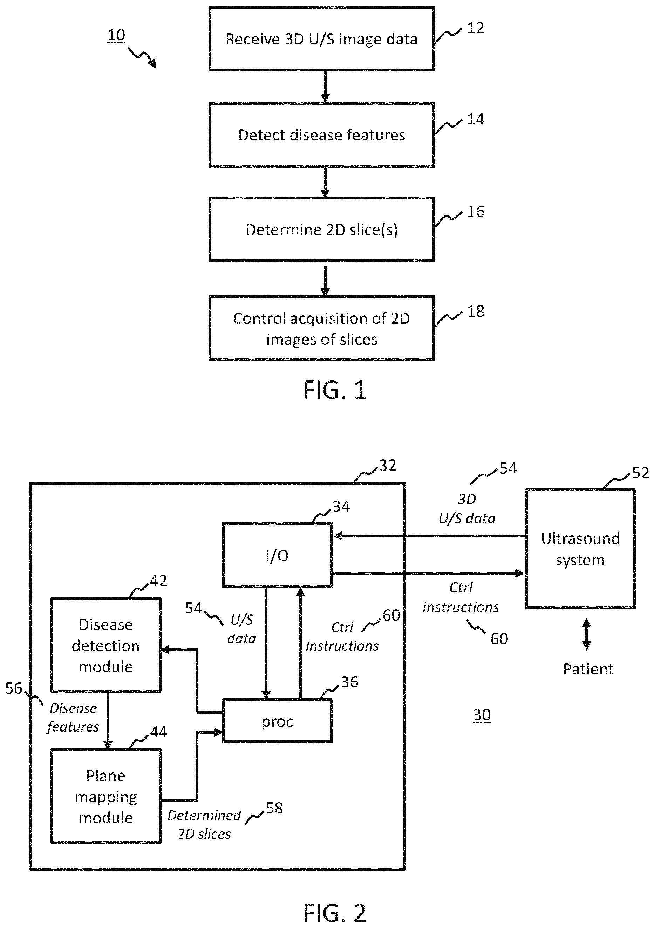

Fig. 1 outlines in block diagram form steps of an example method according to one or more embodiments. The steps will be recited in summary, before being explained further in the form of example embodiments.

The method 10 comprises receiving 12 as input from an ultrasound acquisition system a 3D ultrasound image dataset comprising at least one 3D image frame spanning a 3D field. The method further comprises applying a disease detection module adapted for processing input 3D image frames and detecting 14 one or more suspected disease features therein of a pre-defined set of possible disease features. The method further comprises applying a plane-mapping module adapted to determine 16 for each 3D image frame a set of one or more 2D slices through the 3D field of the image frame based on an output from the disease detection module, for imaging one or more disease regions associated with the detected one or more disease features. The method further comprises generating 18 control instructions for output to the ultrasound acquisition system for causing automated acquisition of the set of one or more 2D slices, for example using a 2D imaging mode, for example using B-mode imaging.

The detected one or more disease features could include physical structures or bodies imaged within the 3D image frame having one or more properties or characteristics associated with a disease state, e.g. a calcified wall section, or a lesion, or a nodule. The detected one or more disease features could additionally or alternatively include classifications of a particular state or characteristic which pertains to a whole anatomical structure (e.g. an organ), or a whole physiological or anatomical system, or to an area of an anatomical structure or system, e.g. abnormal wall motion or abnormal shape of a particular anatomical structure. Thus, the aforementioned disease regions associated with the disease features means disease regions which contain or overlap with at least part of an anatomical structure or area to which the given disease feature relates, or which is relevant for diagnosis of the disease feature. Where the disease feature is a suspicious physical structure or body, the disease region may naturally be, or include, the region containing the structure or body. Where the disease feature is a more general or global classification of a structure or system in the body, then the one or more disease regions may be disease regions which are known to be relevant to diagnosis of the given detected disease feature. Knowledge of such relevant disease regions for a given disease feature may be built into the programming of the plane mapping module, e.g. learned implicitly as part of the training of a machine learning algorithm, or encoded explicitly as part of one or more algorithms employed by the plane mapping module.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention, and also schematically showing the data processing flow in more detail.

The processing device 32 comprises: an input/output 34 and one or more processors 36. The one or more processors 36 are configured to perform a method as follows. The method comprises receiving at the input/output 34, as input from an ultrasound data acquisition system 52 (i.e. an ultrasound scanning system), a 3D ultrasound image dataset 54 comprising at least one 3D image frame spanning a 3D field. The method further comprises applying a disease detection module 42 adapted for processing input 3D image frames and detecting one or more suspected disease features 56 therein of a pre-defined set of possible disease features. The method further comprises applying a plane-mapping module 44 adapted to determine a set of one or more 2D slices 58 through the 3D field of each image based on an output from the disease detection module 42, for imaging one or more disease regions associated with the detected one or more disease features. The method further comprises generating control instructions 60 for output via the input/output 34 to the ultrasound acquisition system 52 for causing automated acquisition of the determined set of one or more 2D slices, for example using a 2D imaging mode, for example using B-mode imaging.

Another aspect of the invention is a system 30 which comprises the processing device 32. Fig. 2 shows the processing device 32 operating within the context of such as system. The system 30 may further comprise the ultrasound acquisition system 52. The system may further comprise a user interface device (not shown), which may be controlled in some embodiments to display a visualization of the acquired 2D image slices.

It is noted that although the disease detection module 42 and the plane mapping module 44 are shown illustratively as separate entities or components within the processing device 32, this is schematic only. In practice, these could be software modules which may both be encoded in programming of a single processor or their functions may be performed in a distributed way by a plurality of processors. Likewise, the item labelled as the one or more processors 36 which perform the method, said method includes steps of calling upon the modules 42 and 44, could be a single processor or multiple processors. In practice the functionality of the disease detection module 42 and the plane mapping module 44 could be encoded on a same processor(s) which executes the high-level method 10 whose steps were outlined above.

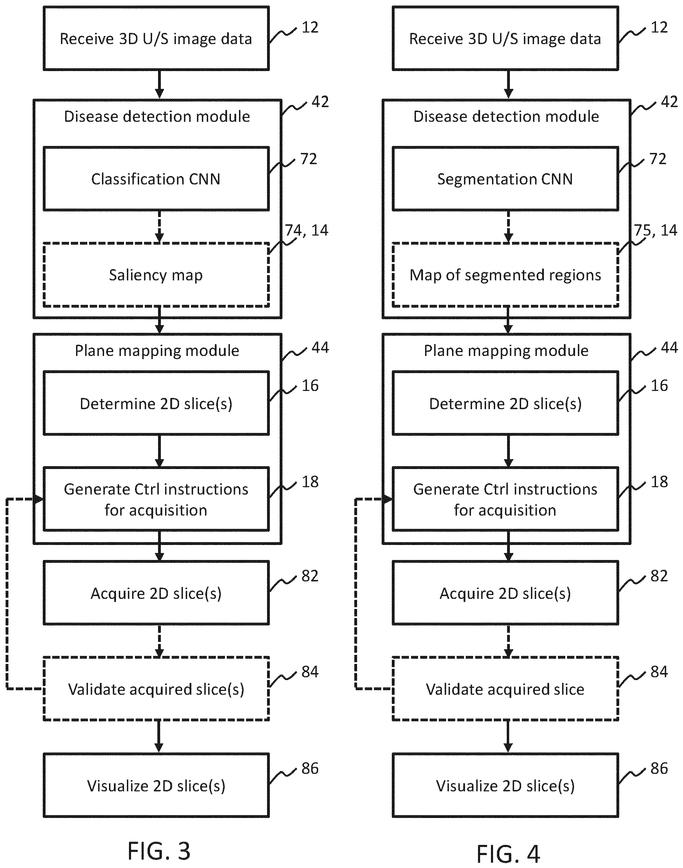

To elucidate the general concepts summarized above, two example implementations of the method according to respective particular groups of embodiments will now be described by way of illustration. These are outlined schematically in Fig. 3 and Fig. 4. It will be appreciated that not all features of these particular groups of embodiments are essential to the inventive concept, and are described to aid understanding and to provide examples to illustrate the more general inventive concepts.

By way of summary, the example implementations of Fig. 3 and Fig. 4 are in most respects similar. They differ mainly in that the embodiment of Fig. 3 proposes, for implementation of the disease detection module 42, to use a convolutional neural network (CNN) trained for several discrete classification tasks and which outputs a classification in respect of a particular disease state, e.g. detection of wall motion abnormality, while the embodiment of Fig. 4 proposes instead, for implementation of the disease detection module 42, to use a CNN trained for one or more segmentation tasks and which outputs at least one segmentation of a suspected structure or body which is indicative of potential disease (e.g. segmentation of calcification).

Both the embodiment of Fig. 3 and the embodiment of Fig. 4 begin with a step 12 of receiving 3D ultrasound image data which includes at least one 3D image frame spanning a 3D field. Optionally, the input 3D ultrasound image dataset comprises a stream of 3D image frames, and wherein the steps of the method are performed in real time with receipt of each 3D image frame.

A disease detection module 42 is then applied to the received 3D ultrasound image data. As briefly mentioned above, the disease detection module is configured for processing the input 3D image frames and detecting one or more suspected disease features therein of a predefined set of possible disease features. To implement the detection of disease features, both the method of Fig. 3 and that of Fig. 4 proposes to utilize a trained machine learning algorithm. More particularly, both the embodiment of Fig. 3 and Fig. 4 proposes to use a trained convolutional neural network (CNN). It is emphasized however that, as a more general principle, there is no necessity for implementing the invention to use a machine learning algorithm. Instead, any other type of algorithm could be utilized, for example other kinds of image processing algorithm which use for example shape detection or model-based segmentation to detect particular structures or to identify elements within an image which might be indicative of particular disease features.

Returning to the present case, the embodiment of Fig. 3 proposes to use a CNN which is trained for deriving at least one discrete classification of the 3D image in relation to one or more particular possible disease features. By way of one example, the CNN could be trained to detect wall-motion abnormality. The CNN can be trained for one or several classification tasks. The output of the CNN in this case therefore is a classification which represents detection of a disease feature, wherein the disease feature may pertain to a plurality of structural elements within the image region or may represent a disease state or characteristic of a generalized system or region. Other (non-limiting) example classifications which the CNN might be trained to derive include: irregular heartbeat, valve dysfunction, abnormal ejection fraction, diastolic dysfunction, systolic heart failure. These examples clearly relate specifically to the cardiac area but, in other embodiments, the CNN may be trained to compute classifications relating to other organs, anatomical regions or physiological systems as appropriate.

Optionally, in the case of use of a classification CNN, the disease detection module 42 may also generate, using data output from the convolutional neural network, a saliency map 74 representing the originating localizations of the model decisions. In relation to implementation of this feature, reference is made to the paper: Visualizing and understanding convolutional networks. Zeiler, MD and Fergus, R. In European Conference on Computer Vision, p. 818-833. 2014.

In other words the final output of the convolution neural network is a classification, or a plurality of classifications. However, as part generating the classification, the neural network may break the input image down into localized areas, and generate sub-classifications for each indicative of a saliency of the localized area to the overall classification which the CNN is trained to determine. From this saliency data, a saliency map can be computed, either by the CNN itself or by the disease detection module, based on information output from the CNN in combination with the classification(s). In other words, in determining the disease feature, the CNN generates saliency data for the 3D field of the input image in relation to the disease feature, and derives a discrete classification of the 3D image in relation to the disease feature based on this information, and wherein a saliency map spanning the 3D field and indicative of the saliency information is used as the 3D spatial map of disease regions.

Looking by way of comparison to the embodiment of Fig. 4, here the convolutional neural network 72 is trained to perform one or several segmentation tasks. Thus here, the detection of the one or more disease features by the disease-detection module 42 comprises segmenting and classifying one or more spatial regions as suspicious regions. By way of purely illustrative example, the segmentation tasks might include one or more of:
- Segmentation of a heart valve, for example to detect a leaflet defect, such as flailing leaflets, prolapse, or tenting. This might be done by shape analysis for example.
- Segmentation of the left ventricular outflow tract (LVOT), and for example detection of obstruction of the LVOT; and
- Ventricle segmentation, for example for detection of dilated heart (e.g. dilated cardiomyopathy).

Optionally, in the case of use of a segmentation CNN 72, a 3D spatial map may further be generated and output by the disease detection module 42 which comprises a map 75 of the segmented suspicious regions.

Thus, as a more general principle, it can be seen that an optional feature for both the embodiment of Fig. 3 and Fig. 4 is that the disease detection module 42 generates for the at least one input 3D image frame a 3D spatial map 74, 75 of disease regions within the 3D field of the image frame corresponding to the disease features. This could be a map of segmented regions 75 in the case of the embodiment of Fig. 4, or could be a saliency map 74 in the case of the embodiment of Fig. 3.

The output of the disease detection module 42 is fed into a plane mapping module 44.

The plane mapping module 44 performs an optimization step that computes an optimum set of one or more 2D slices through the 3D field of the image frame for imaging one or more disease regions associated with the detected one or more disease features. In other words, an optimum acquisition protocol is determined, including a plurality of 2D slices and optionally beam forming parameters and frame rate that is best suited to validate any potential finding detected by the disease detection module.

In the present cases, the plane-mapping module 44 is adapted for receiving as input the 3D map of detected disease regions within a 3D field, and is adapted for determining a set of one or more 2D slices through the 3D field intersecting with the disease regions, in dependence upon the map.

In the case of a saliency map 74 (Fig. 3) being generated, the saliency map can be used as the 3D spatial map of disease regions. One approach for example would be to compute slices that best cover all regions with high saliency response. In other words, the plane mapping module 44 is adapted to determine the set of one or more 2D slices based on fitting planes of maximum saliency through the saliency map. A plane of maximum saliency means for example a plane which plots a planar path of maximum saliency, i.e. a plane which contains/covers maximum aggregate saliency values.

Optionally, the determined planes may be determined such that they meet a further one or more constraints. With reference to the further one or more constraints, these may depend in part on the specific disease features to which the disease regions correspond. The further one or more constraints may comprise a requirement to minimize the number of 2D slices, while still intersecting all of the regions. The further one or more constraints may comprise one or more allowable ranges for 2D slice plane orientation relative to one or more directions.

An example implementation in some examples could use weighted saliency responses and determine a closest standard, user-defined, or automatically-defined anatomical plane to derive the optimum slice. The automatically-defined anatomical planes can be derived, as noted above, by fitting planes through maximum saliency (e.g. a maximum intensity projection aligned along certain anatomical landmarks).

In the case (Fig. 4) that the disease detection module 42 generates a 3D spatial map of segmented regions 75, the 3D spatial map of segmented suspicious regions could be used to derive the 2D planes. One approach for example would be for the plane mapping module to perform a spatial fitting of planes to the one or more disease regions in the map, to determine a set of one or more 2D slices which intersect all of the regions, and optionally which meet a further one or more constraints (see discussion above).

If no 3D map of disease regions is generated (i.e. in variance to the embodiments of Fig. 3 and Fig. 4), another approach to determining the planes might be to utilize guidelines or protocols derived from a database or a prediction model, i.e. selecting the anatomical plane that is typically best suited to detect the possible diseases connected to the detected disease feature(s). For example, the plane-mapping module 44 may be adapted to use a look-up table to select a pre-determined plane based on the one or more detected disease features.

In both the method of Fig. 3 and the method of Fig. 4, the method further comprises, after having determined the 2D slices, generating control instructions 18 for output to an ultrasound acquisition system for causing automated acquisition of the determined set of one or more 2D slices, using for example B-mode imaging. These control instructions may be generated by the plane mapping module 44, or by another module or routine of the one or more processors of the processing device 32 which implements the method. The ultrasound acquisition system receives the instructions and acquires 82 the 2D slices in accordance with the instructions.

The input to the ultrasound acquisition system is the set of control instructions. At the point of receiving the control instructions, the ultrasound acquisition system may still be in use by the operator for acquiring image data. Optionally, the acquisition of the new 2D slices (e.g. in B-mode) may be done in the background, optionally informing the user, using a prompt presented on a user interface display, that the additional acquisition is in progress and indicating that there is a need therefore to hold the probe in position. In some examples, the control instructions may be adapted to cause the ultrasound acquisition system to interleave acquisition of the set of one or more 2D slices with any other acquisition sequence which the ultrasound acquisition system is currently performing.

In some examples, the method further comprises, after determining the set of 2D slices, controlling a user interface to generate a user-perceptible prompt requesting approval to acquire the set of 2D slices, and wherein the generating of the control instructions is performed only responsive to receipt from the user interface of a user input indicative of approval. This might be generated on a user interface of the ultrasound acquisition system in some examples. For example, the recording of additional slices may be triggered by a user interface event and an optional preview of proposed acquisition protocols or pictograms. This could be e.g. a dialog box that asks the operator for permission to acquire the slices and obligatory user input in form of an OK button press, a foot pedal, a button, etc. to release the acquisition of additional slices.

With regards to the generating of control instructions which will lead to the acquisition of the specifically derived set of 2D planes, a dedicated module may be included in the processing device for the purpose of generating such control instructions. The control instructions may indicate acquisition parameters which the ultrasound acquisition system should use to acquire the one or more 2D slices. For instance, these could include beam forming parameters. They could also include other parameters specifying for example one or more of: scan line density, transmit frequency, and receive filtering, for optimizing the image quality for the desired scanned region.

Of course, an ultrasound imaging system 52 may typically include a local beamform controller which is operable to control beamform parameters to acquire a selected one or more planes, for example defined relative to a co-ordinate system of the field of view of the imaging probe. Therefore the control instructions might in some examples simply provide an indication of the one or more planes within the imaging field of view which should be acquired and wherein the necessary acquisition parameters, including beamforming parameters, to acquire those planes are determined locally by the ultrasound acquisition system 52.

The ultrasound acquisition system thus receives the instructions and acquires the 2D slices 82 in accordance with the instructions.

Preferably, the control instructions are adapted to control acquisition of 2D slices which are of higher spatial resolution than the input 3D ultrasound image data. In order to achieve a minimum temporal resolution, 3D imaging is typically limited in its spatial resolution for each 3D frame. Therefore, by acquiring 2D images of each suspected disease region, the diagnostic analysis can be done with imagery which is of higher spatial resolution.

Optionally, there may be implemented an additional step to check or validate 84 the acquired slices against the planned optimal slices determined by the plane mapping module 44, and whose indication was output from the plane mapping module.

This may make use of a validation module which performs a comparison of a given newly acquired 2D image with a 2D slice extracted from 3D imaging data corresponding to the determined 2D plane intended to be acquired. It can therefore be determined whether the acquired image actually matches the intended plane to be imaged. For instance, the patient may have moved between determining the plane and capturing the 2D image, which could lead to the acquired image frame not matching the intended slice through the anatomy.

For example, the comparison could be done by computing a similarity measure between the respective slice from the 3D volume (e.g. with coarser resolution) and the acquired 2D image (e.g. with finer resolution). The same features should appear in both images, for example the same anatomical landmarks should be present, with common position and orientation. In other words, in some examples, once the 2D slice(s) to-be-acquired have been determined, corresponding 2D slices through the 3D dataset may be extracted (e.g. using multiplanar reformatting) and these may then be compared with the newly acquired 2D images to check a degree of matching between the two.

Additionally or alternatively, the validation may for example make use of a plane predictor module which is applied to the acquired 2D images generated by the ultrasound acquisition system, and is adapted to generate, as output, a prediction of an anatomical plane to which each acquired 2D image corresponds. This may for example be a plane regression module which has the task of estimating for a given acquired 2D image, which plane the image represents through the imaged anatomical structure. The results of the plane predictor module can then be compared with the planned or intended 2D slice, and it can therefore be determined whether the acquired image actually matches the intended plane to be imaged.

For example, a plane predictor module could be implemented by training a regression CNN with pairs of 2D images and respective known plane parameters. It has been found by the inventors that such a predictor can be made even more stable if, in training, instead of using the whole 2D image, the CNN is trained with detected anatomical contours. This effectively creates a hand-crafted feature bottleneck. An example algorithm following this approach (in a slightly different domain of skeletal x-ray) has been published as: Krönke et al. CNN-based pose estimation for assessing quality of ankle-joint X-ray images. SPIE Medical Imaging 2022

In a further step, the method preferably comprises controlling a user interface to generate a visual output 86 representative of the acquired set of one or more 2D slices. These could be visualized either immediately or upon request by the user for example.

In some embodiments, optionally, in addition to determining one or more slices that would help support or confirm a suspected disease finding, one or more slices could be acquired that might contradict or negate the suspicion. This may improve confidence in diagnosis with an unbiased view on the suspicious finding.

In some embodiments, an additional step may be performed in which the acquired 2D image slices are fed as a further input to the disease detection module 42. The result of this can help confirm or reinforce the previous disease detection findings. For example, if the result of the further application of the disease detection module is a negative finding (no disease feature detected), then the acquired image slice might be discarded. This reduces false positives.

In some embodiments, the acquired one or more planes for one or more of the possible disease features might include standardized plane(s) which have been determined in advance to represent an optimum or optimized anatomical view for analyzing the given disease features. A look-up table could be used to look up the standard plane(s) for a given feature. One way of generating the standard planes might be to, in advance, execute a training phase in which a trained machine learning module receives as input multiple saliency-map derived planes (from different exams and patients) and delivers as output a rule-based plane mapping, e.g. by selecting three plane-defining landmarks. This embodiment brings the chance of improving the general plane portfolio. For specific diseases, novel "standard" planes could be defined, for instance.

One particularly advantageous application for embodiments of this invention is in the field of cardiac imaging, for ultrasound examinations for the human heart. However, application is not at all restricted to this field. It is generally applicable to any imaging modality. It may find particularly advantageous application for non-ionizing imaging modalities. It may find particularly advantageous application for imaging modalities that allow acquisition of images with different levels of quality/resolution using a constant geometrical set-up, e.g., optical coherence tomography (OCT). Furthermore, the body part of interest can be any part of the human body and is not restricted to the heart. The invention could be applied for instance, and by way of non-limiting example, to fetal scanning, pathology, or veterinary applications.

As mentioned previously, the invention can be embodied in software form. Thus another aspect of the invention is a computer program product comprising code means configured, when run on a processor, to cause the processor to perform a method in accordance with example or embodiment of the invention described in this document, or in accordance with any claim of this patent application.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing arrangement can be implemented. The processing device may include a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method, comprising:
receiving (12) as input from an ultrasound acquisition system a 3D ultrasound image dataset comprising at least one 3D image frame spanning a 3D field;
applying a disease detection module adapted for processing input 3D image frames and detecting (14) one or more suspected disease features therein of a pre-defined set of possible disease features;
applying a plane-mapping module adapted to determine (16) for each 3D image frame a set of one or more 2D slices through the 3D field of the image frame based on an output from the disease detection module, for imaging one or more disease regions associated with the detected one or more disease features; and
generating control instructions (18) for output to the ultrasound acquisition system for causing automated acquisition of the set of one or more 2D slices.

2. The method of claim 1, wherein the input 3D ultrasound image dataset comprises a stream of 3D image frames, and wherein the steps of the method are performed in real time with receipt of each 3D image frame.

3. The method of claim 1 or 2, wherein the plane-mapping module is adapted to use a look-up table to select a pre-determined plane based on the one or more detected disease features.

4. The method of claim 1 or 2,
wherein the disease detection module is adapted to generate for the at least one 3D image frame a 3D spatial map of disease regions within the 3D field of the image frame corresponding to the disease features; and
wherein the plane-mapping module is adapted for receiving as input a 3D map of detected disease regions within a 3D field, and is adapted for determining a set of one or more 2D slices through the 3D field intersecting with the disease regions, in dependence upon the map.

5. The method of claim 4, wherein, the plane mapping module is adapted to perform a spatial fitting of planes to the one or more disease regions, to determine a set of one or more 2D slices which intersect all of the regions, and optionally which meet a further one or more constraints.

6. The method of claim 4 or 5, wherein, for at least a subset of the pre-defined set of disease features, the detection of the disease feature by the disease-detection module comprises segmenting and classifying one or more spatial regions as suspicious regions, and wherein the 3D spatial map output by the disease detection module comprises a map of the segmented suspicious regions.

7. The method of any of claims 4-6, wherein, for at least a subset of the pre-defined set of disease features, the detection of the respective disease feature by the disease-detection module comprises computing a 3D saliency map spanning the 3D field in relation to the disease feature, and deriving a discrete classification of the 3D image in relation to the feature based on the saliency map, and wherein the saliency map is used as the 3D spatial map of disease regions.

8. The method of claim 7, wherein the plane mapping module is adapted to determine the set of one or more 2D slices based on fitting planes of maximum saliency through the saliency map.

9. The method of any of claims 1-8, wherein the control instructions are adapted to cause the ultrasound acquisition system to interleave acquisition of the set of one or more 2D slices with any other acquisition sequence which the ultrasound acquisition system is currently performing.

10. The method of any of claims 1-9, wherein the disease detection module comprises at least one trained machine learning algorithm, and preferably a convolutional neural network (CNN).

11. The method of any of claims 1-10, wherein the control instructions are adapted to control acquisition of 2D slices which are of higher spatial resolution than the input 3D ultrasound image data.

12. The method of any of claims 1-11, wherein the method further comprises receiving the acquired set of one or more 2D slices; and controlling a user interface to generate a visual output representative thereof.

13. The method of any of claims 1-12, wherein the method further comprises, after determining the set of 2D slices, controlling a user interface to generate a user-perceptible prompt requesting approval to acquire the set of 2D slices, and wherein the generating of the control instructions is performed only responsive to receipt from the user interface of a user input indicative of approval.

14. A processing device (32) comprising:
an input/output (34); and
one or more processors (36) configured to perform a method comprising:
receiving at the input/output, as input from an ultrasound acquisition system (52), a 3D ultrasound image dataset (54) comprising at least one 3D image frame spanning a 3D field;
applying a disease detection module (42) adapted for processing input 3D image frames and detecting one or more suspected disease features (56) therein of a pre-defined set of possible disease features;
applying a plane-mapping module (44) adapted to determine a set of one or more 2D slices (58) through the 3D field of each image based on an output from the disease detection module, for imaging one or more disease regions associated with the detected one or more disease features; and
generating control instructions (60) for output via the input/output to the ultrasound acquisition system (52) for causing automated acquisition of the set of one or more 2D slices.

15. A system (30) comprising:
an ultrasound acquisition system (52); and
the processing device (32) of claim 14, operatively coupled to the ultrasound acquisition system.
